# EUROPEAN PATENT APPLICATION

(11) **EP 1 491 629 A1**
(43) Date of publication of application: **29.12.2004**
(21) Application number: 03744032.8
(22) Date of filing: 10.03.2003
(51) Int. Cl.: C12N 15/09, C12Q 1/68

(54) **NOVEL METHOD OF HIGHLY SENSITIVE NUCLEIC ACID ANALYSIS**

(30) Priority: 08.03.2002 JP 2002063960; 07.03.2003 JP 2003061958
(71) Applicant: Matsumoto, Kazuko, Setagaya-Ku, Tokyo 155-0032 (JP); Mitsubishi Rayon Co., Ltd., Tokyo 108-8506 (JP)
(72) Inventor: MATSUMOTO, Kazuko, Setagaya-ku, Tokyo 155-0032 (JP); YUAN, Jingli, Liaoning Province (CN)
(74) Representative: HOFFMANN EITLE
(86) International application number: PCT/JP2003/002775
(87) International publication number: WO 2003/076615

(57) **Abstract**

It is intended to provide a method of highly sensitively analyzing SNPs. More specifically, it is intended to provide an SNP analysis method whereby a large amount of SNPs can be quickly analyzed using a DNA sample in a trace amount. An SNP analysis method by the invader method with the use of a FRET probe having a luminescent dye and a quencher characterized by using, as the luminescent dye in the FRET probe, a rare earth fluorescent complex label made of a rare earth element such as europium or terbium. A composition for controlling fluorescent luminescence which comprises a combination of a specific rare earth fluorescent complex label with a specific fluorescent quencher. A highly sensitive labeling probe characterized by using a rare earth fluorescent complex label as a luminescent dye and a fluorescent quencher label as a quencher in a highly sensitive labeling probe such as a FRET probe and an SNP analysis kit containing the same.

SEQUENCE LISTING

<110> Matsumoto, Kazuko

<120> A New Method for Nucleotid Analisys

<130> JA924409

<150> JP2002-063960
<151> 2002-03-08

<150> JP2003-061958
<151> 2003-03-07

<160> 10
<170> PatentIn version 3.1

<210> 1
<211> 44
<212> DNA
<213> Artificial Sequence
<220>
<223> Target DNA

<400> 1
ggaagaggag gagggtgctc aggaggagcg ggaggacact gtgt 44

<210> 2
<211> 27
<212> DNA
<213> Artificial Sequence
<220>
<223> Primary Probe

<400> 2
aacgaggcgc acccctcctc ctcttcc 27

<210> 3
<211> 30
<212> DNA
<213> Artificial Sequence
<220>
<223> Secondary probe

<400> 3
acacagtgtc ctcccgctcc tcctgagcac 30

<210> 4
<211> 39
<212> DNA
<213> Artificial Sequence
<220>
<223> FRET probe

<400> 4
tcttgtctcg gtttttccga gacaagagtg cgcctcgtt 39

<210> 5
<211> 22
<212> DNA
<213> Artificial Sequence

<220>
<223> Target DNA

<400> 5
gctggaggga tgatactttg ca 22

<210> 6
<211> 32
<212> DNA
<213> Artificial Sequence

<220>
<223> Molecular Beacon

<400> 6
ccaagcgcaa agtatcatcc ctccaggctt gg 32

<210> 7
<211> 24
<212> DNA
<213> Artificial Sequence

<220>
<223> Target DNA

<400> 7
ccatggtgtc tgtttgaggt tgct 24

<210> 8
<211> 24
<212> DNA
<213> Artificial Sequence

<220>
<223> Target DNA

<400> 8
ccatggtgtc tgtttcaggt tgct 24

<210> 9
<211> 24
<212> DNA
<213> Artificial Sequence

<220>

<223> Target DNA

<400> 9

ccatggtgtc tgtttaaggt tgct                                              24

<210> 10

<211> 24

<212> DNA

<213> Artificial Sequence

<220>

<223> Target DNA

<400> 10

ccatggtgtc tgttttaggt tgct                                              24

## Description

### Technical Field

The present invention relates to a highly sensitive SNP analysis method characterized by using a rare earth fluorescent complex labeling agent as a luminescent dye, more specifically relates to a method of a highly sensitive single nucleotide polymorphism analysis characterized by using a rare earth fluorescent complex labeling agent as a luminescent dye and measuring fluorescence luminescence based on a time-resolved fluorescence assay. In addition, the present invention relates to a composition for controlling fluorescence luminescence comprising a combination of a specific rare earth fluorescent complex labeling agent and a specific fluorescent quencher labeling agent. Furthermore, the present invention relates to a highly sensitive labeled probe characterized in that, in a highly sensitive labeled probe such as a FRET probe, a rare earth fluorescent complex labeling agent is usedas a luminescent dye and a fluorescent quencher labeling agent is used as a quenching substance, and a kit for an SNP analysis comprising the same.

### Background Art

Conventionally, as analysismethods for single nucleotide polymorphism (abbreviated as SNP) of human genome DNA, there are TaqMan PCR method, MALDI-TOF/MS method, DNA chip method, Invader method, a method using RCA (rolling circle amplification) and the like. These methods have their own characteristics and are used in various SNP analyses.

The TaqMan PCR method is an SNP analysis method using PCR reaction and has an advantage of being composed of a small number of analysis steps (T. Morris et al., J. Clin. Microbiol., 1996, 34, 2933., K. J. Livak et al., Genet. Anal., 1999, 14, 143). However, this method has a disadvantage of high cost for labeled probe preparation, because it needs two kinds of fluorescent labeled probes to analyze one kind of SNP.

The MALDI-TOF/MS (Matris assisted laser desorption-time of flight/mass spectrometry) method has an advantage of using one primer without label for one SNP analysis (L, A. Haff et al., Genome Res., 1997, 7, 378. P. Ross et al., Nat. Biotechnol., 1998, 16, 1347), however, it has a disadvantage in that the number of operation steps is very large, such as PCR amplification of a target region, purification of a PCR product, primer extension reaction, purification of a primer extension reaction product, spotting of a sample for mass measurement, mass analysis and the like.

The DNA chip method has an advantage in that a large amount of SNPs can be analyzed at one time (D. G. Wang et al., Science, 1998, 280, 1077., J. G. Hacia et al., Nat. Genet., 1999, 22, 164., P. N. Gilles et al., Nat. Biotechnol., 1999, 17, 365). This method needs PCR before hybridization is conducted on a DNA chip, therefore a large number of PCRs must be conducted in advance of analyzing a large amount of SNPs.

The Invader method is composed of a small number of SNP analysis steps, and does not need PCR and a fluorescent labeled allele specific oligonucleotide, and only needs an addition of a flap that is common among each of allele-specific oligonucleotides.

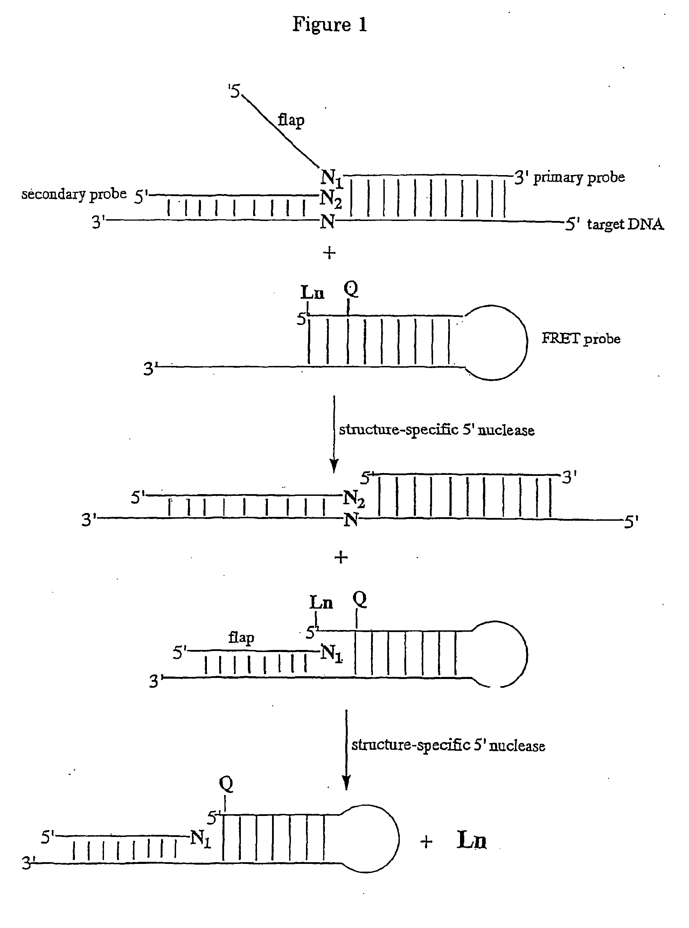

Fig. 1 shows an overview of a single nucleotide polymorphism analysis method by the Invader method as a diagram. This method is a method of determining the existence of a nucleotide which is an SNP at the position of "N" in a target DNA as shown in Fig. 1. Due to this, a reporter probe (primary probe), an invader probe (secondary probe), a FRET probe for detection and a florescence resonance energy transfer probe (FRET probe) are used. The reporter probe contains a complementary nucleotide sequence to the 5' side of the SNP position of the target DNA and further contains a nucleotide sequence region called "flap". The nucleotide sequence of this flap region is a complementary nucleotide sequence to the nucleotide sequence of the 3' side of the FRETprobe. Inaddition, the invader probe has a complementary nucleotide sequence in the 3' side of the target DNA. The nucleotide "N" of the target DNA at the SNP position (the position indicated by "N" in Fig. 1) is a nucleotide of single nucleotide polymorphism site and any one of A, T, G and C. The nucleotide "N₁" of the reporter probe is a nucleotide that can form a normal base pair with N, and the "N₂" of the invader probe is an arbitrary nucleotide.

The FRET probe is a probe that contains a single-stranded region at the 3' side where the flap region binds, and at the 5' side of the FRET probe, a part or the whole of it forms a double strand. Although, in Fig. 1, the whole 5' side is shown to form a double strand, the whole region does not necessarily form a double strand. However, here the explanation is made in accordance with Fig. 1 for explanation. Furthermore, a luminescent dye "Ln" and a quenching substance "Q" are bound to the 5' side of the FRET probe. When the quenching substance "Q" exists within a fixed distance from the luminescent dye "Ln", the luminescence from the luminescent dye is quenched and the luminescence cannot be observed.

In the Invader method, a reporter probe and an invader probe bind to a target DNA first, and at this time, the nucleotide "N₂" of the invader probe is inserted in the middle of the base pair of the nucleotide "N₁" of the reporter probe and the nucleotide "N" of the target DNA as if it is an invader. This situation is shown in the uppermost part of Fig. 1.

If a cleavage enzyme (structure-specific 5' nuclease), which specifically works in this type of situation where 3 nucleotides such as these are bound with one another, this cleavage enzyme cleaves the reporter probe at the position of the nucleotide "N₁" and a fragment containing only the flap region is formed. Then, this flap region binds to the 3' side of the FRET probe. This situation is shown in the middle part of Fig. 1. At this time, the edge part of the flap region containing the nucleotide "N₁" is inserted in the middle of the double-stranded part of the FRET probe. This situation is similar to that of the above-mentioned invader probe and becomes a specific cleavage position due to the cleavage enzyme. Then, the cleavage enzyme cleaves at the region where the 5' end part of the FRET probe is broken in by the flap region.

The luminescent dye "Ln" is bound to the 5' side of the cleaved FRET probe and the luminescent dye "Ln" is separated from the quenching substance "Q" by this cleavage. As a result, the luminescence of the luminescent dye "Ln" becomes observable. This situation is shown in the lowermost part of Fig. 1. The luminescent dye "Ln" is released from the quenching substance "Q" and shows its original luminescence.

In the case where the nucleotide "N₁" of the reporter probe cannot form a normal base pair with the nucleotide "N" of the target DNA, the invader-like situation is not attained, therefore, the cleavage by the cleavage enzyme is not caused. Accordingly, whether or not the nucleotide "N" of the target DNA is a nucleotide that can form a normal base pair with the nucleotide "Nᵢ" of the reporter probe can be judged by the luminescence.

As mentioned above, in the Invader method, not only does a target DNA not need to be labeled but also a reporter probe and an invader probe do not need to be labeled and what needs to be labeled is only a FRET probe. In addition, the FRET probe is associated only with the nucleotide sequence of the flap region and not associated in particular with the nucleotide "N₁" of the flap region. Therefore, because the FRET probe is not at all affected by the nucleotide sequence of a target DNA and is based on the nucleotides of a flap region determined arbitrarily without regard for the target DNA, there is an advantage in that a large-scale production is possible so as to reduce the costs of probe production to a large degree.

However, because the measurement sensitivity of a conventional method is not enough, several tens of nanograms of genome DNA is necessary for the analysis of one SNP, therefore it is disadvantageous in that a large amount of genome DNAs is necessary for the analysis of a large amount of SNPs.

### Disclosure of the invention

In the view of the foregoing circumstance, the present invention intends to provide a highly sensitive SNP analysis method and to provide an SNP analysis method, whereby a large amount of SNPs can be quickly analyzed with a trace amount of a target DNA. In addition, the present invention intends to provide a highly sensitive luminescent dye, and more specifically, a fluorescent color developing composition comprising a highly sensitive luminescent dye and a quenching substance. Furthermore, the present invention intends to provide a highly sensitive labeled probe such as a FRET probe composed of a highly sensitive color developing dye and a quenching substance and the highest sensitive SNP analysis method by a time-resolved fluorescence assay using the this FRET probe in a nucleotide SNP analysis method.

### Brief Description of the Drawings

Fig. 1 schematically shows an overview of a SNP analysis method using the Invader method.
Fig. 2 schematically shows an overview of an example of a FRET probe of the present invention.
Fig. 3 schematically shows the SNP analysis method of the present invention, using as an example the case where a FRET probe composed of BPTA-Tb³⁺ and Dabcyl is used.
Fig. 4 shows an absorbance spectrum of a fluorescent quencher labeling agent, Dabcyl, of the present invention and a fluorescence luminescence spectrumof a rare earth fluorescent complex labeling agent, BPTA-Tb³⁺, of the present invention. The horizontal axis of Fig. 4 is wavelength (nm), the vertical axis on the left shows fluorescence intensity and the vertical axis on the right shows absorbance.
Fig. 5 shows changes in fluorescence intensity accompanying the concentration changes of a target DNA in the method of the present invention. The horizontal axis of Fig. 5 is concentration (pM) of a target DNA and the vertical axis shows fluorescence intensity.

### Best Mode for Carrying Out the Invention

Conventionally, some of rare earth fluorescent complex labeling agents have been used for a time-resolved fluorescence assaybecause they have strong fluorescence intensity, although they are highly dependent on the structure or composition of a ligand. However, these take advantage of the strong fluorescence intensity of a rare earth fluorescent complex labeling agent; the control of luminescence by a combination of a rare earth fluorescent complex labeling agent and a fluorescent quencher labeling agent was not developed. In the present invention, it was found that the luminescence can be controlled by a combination of a specific rare earth fluorescent complex labeling agent and a specific fluorescent quencher labeling agent.

In other words, the present invention relates to an SNP analysis method characterized by using a rare earth fluorescent complex labeling agent as the luminescent dye of a highly sensitive labeled probe in an SNP analysis method, either the Invader method in which a FRET probe containing a fluorescent dye and a quenching substance is used, or the Molecular beacon method in which a probe with a hairpin structure is used, or the like. More specifically, the present invention relates to an SNP analysis method characterized by using a rare earth fluorescent complex labeling agent comprising any one of the ligands represented by the following general formulae (1) to (6). Further specifically, the present invention relates to an SNP analysis method characterized by using any one of the fluorescent quencher labeling agents represented by the following general formulae (7) to (9) as a quenching substance. Further specifically, the present invention relates to a highly sensitive SNP analysis method characterized by using a time-resolved fluorescence assay as a fluorescence assay method.

In addition, the present invention relates to a composition for controlling fluorescence luminescence comprising a combination of a specific rare earth fluorescent complex labeling agent and a specific fluorescent quencher labeling agent. More specifically, the present invention relates to a composition for controlling fluorescence luminescence comprising one or more rare earth fluorescent complex labeling agents composed of a ligand represented by the following general formulae (1) to (6) and a rare earth element, and one or more fluorescent quencher labeling agents composed of the following general formulae (7) to (9). 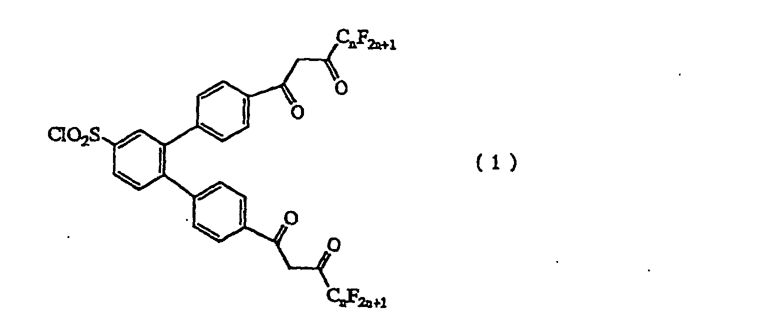 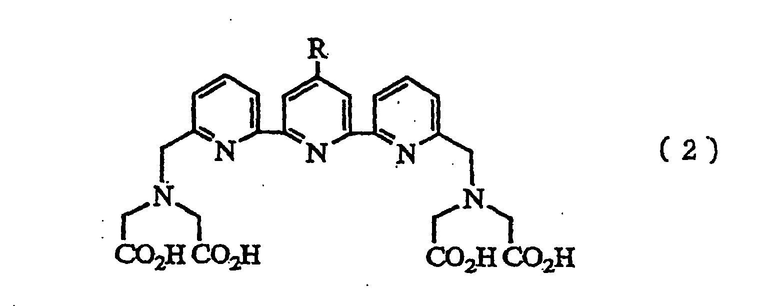 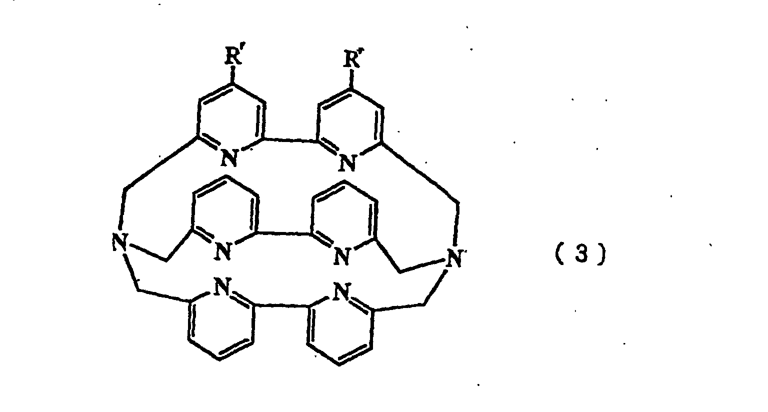 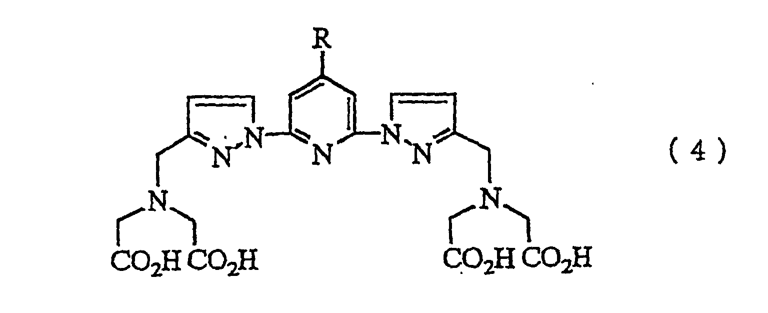 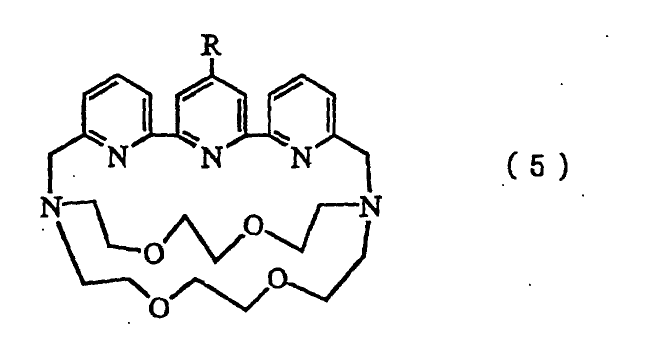 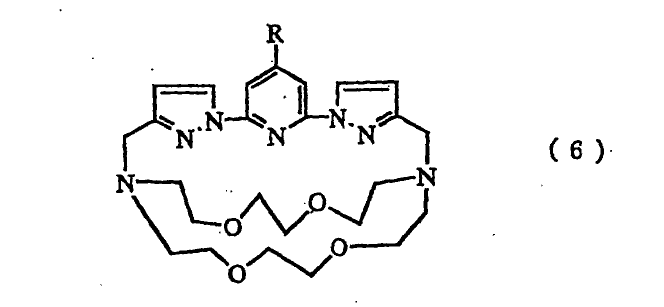 (In the formulae (1) to (6), n represents an integer of 1 to 4, R represents an allyl group having a substituent group and R' represents an amino group, a hydroxyl group, a carboxyl group, a sulfonate group or an isothiocyanate group.) 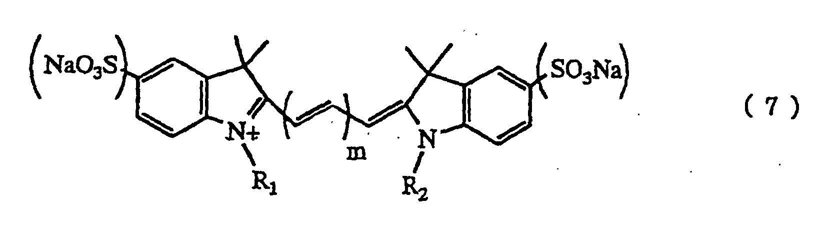 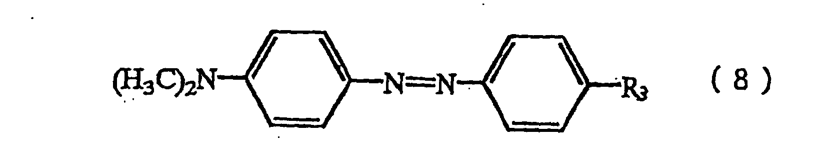 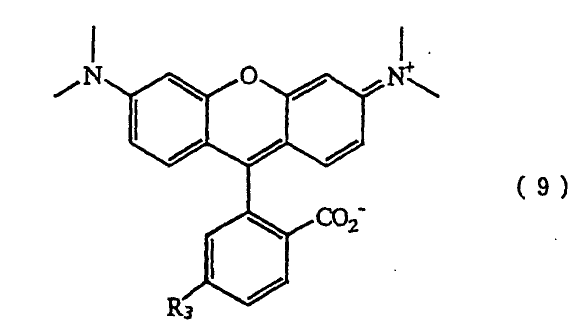 (In the formulae (7) to (9), m represents an integer of 1 to 4, either R₁ or R₂ represents a linker group for immobilization on a carrier or a nucleic acid, the other represents a hydrogen atom or an alkyl group, and R₃ represents a linker group for immobilization on a carrier or a nucleic acid.)

In addition, the present invention relates to a highly sensitive labeled probe such as a FRET probe using a rare earth fluorescent complex labeling agent as an luminescent dye and a fluorescent quencher labeling agent as a quenching substance, and more specifically, relates to a highly sensitive labeled probe using one or more of rare earth fluorescent complex labeling agents comprising a ligand represented by the above-mentioned general formulae (1) to (6) and a rare earth ion as a luminescent dye, and using one or more of fluorescent quencher labeling agents comprising the above-mentioned general formulae (7) to (9) as a quenching substance.

Furthermore, the present invention relates to a composition for an SNP analysis or a kit for an SNP analysis comprising the above-mentioned highly sensitive labeled probe of the present invention.

The present invention is characterized in that a rare earth fluorescent complex labeling agent "Ln" as a luminescent dye in Fig. 1, more specifically, a rare earth fluorescent complex labeling agent comprising a ligand represented by the above-mentioned general formulae (1) to (6) and a rare earth ion, and a fluorescent quencher labeling agent "Q" as a quenching substance, more specifically, a fluorescent quencher labeling agent comprising the above-mentioned general formulae (7) to (9), are used.

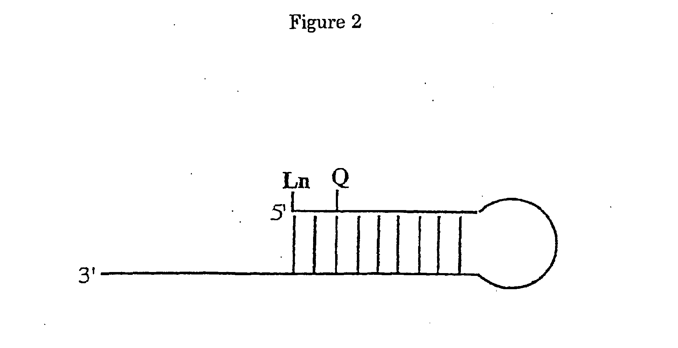

Fig. 2 shows an example of the FRET probe of the present invention. In this example, a rare earth fluorescent complex labeling agent "Ln" is indicated at the 5' end of the probe, however, the FRET probe of the present invention is not limited thereto. The FRET probe of the present invention may be any configuration as long as the fluorescent quencher labeling agent "Q" is located within a distance enough to quench the fluorescence by the rare earth fluorescent complex labeling agent "Ln", and the rare earth fluorescent complex labeling agent "Ln" or the fluorescent quencher labeling agent "Q" is located at a position where it is cleaved by a cleavage enzyme and released in the invader state, the rare earth fluorescent complex labeling agent "Ln" and the fluorescent quencher labeling agent "Q" being separated by the cleavage and the distance between them being kept large enough to observe luminescence, and the position is not limited to the one illustrated in Fig. 2.

To illustrate the present invention more concretely, an explanation is made using a terbium complex fluorescent labeling agent (hereinafter abbreviated as BPTA-Tb³⁺) composed of a terbium ion and an organic ligand N,N,N¹,N¹-[2,6-bis(3'-aminomethyl-1'-pyrazolyl)-4-phenylpyr idine] tetrakis (acetic acid) (a ligand represented by the above-mentioned general formula (2) wherein the substituent group R is a phenyl group, hereinafter abbreviated as BPTA) as a rare earth complex labeling agent and a compound represented by the general formula (9) (hereinafter abbreviated as Dabcyl) as a fluorescent quencher labeling agent. The synthesis of BPTA and its N-hydroxysuccinateimide monoester (abbreviated as NHS-BPTA) was carried out according to the method reported by the present inventors (J. Yuan, G. Wang, K. Majima, K. Matsumoto, Anal. Chem., 2001, 73, 1869). The chemical structure of BPTA-Tb³⁺ is shown below.

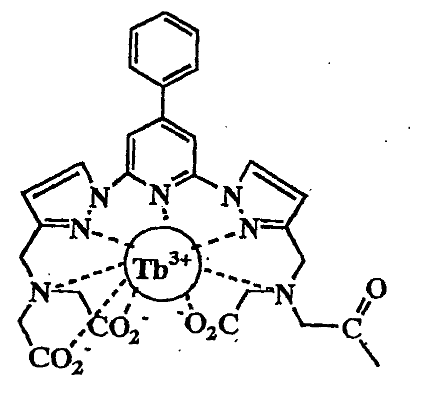

In addition, as DNAoligonucleotides, the followings were used. As the target DNA, the following 3'TGTGTCACAGGAGGGCGAGGAGGACTCGTGGGAGGAGGAGAAGG5' was used, and as the reporter probe, the following 5'AACGAGGCGCACCCCTCCTCCTCTTCC3' was used, and as the invader prove, the following 5'ACACAGTGTCCTCCCGCTCCTCCTGAGCAC3' was used. As the FRET probe, to which the rare earth fluorescent complex labeling agent HPTA-Tb³⁺ and the fluorescent quencher labeling agent Dabcyl were immobilized, the following 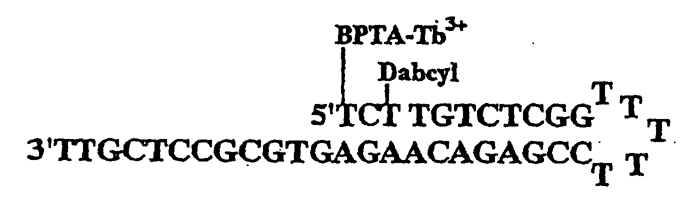 was used.

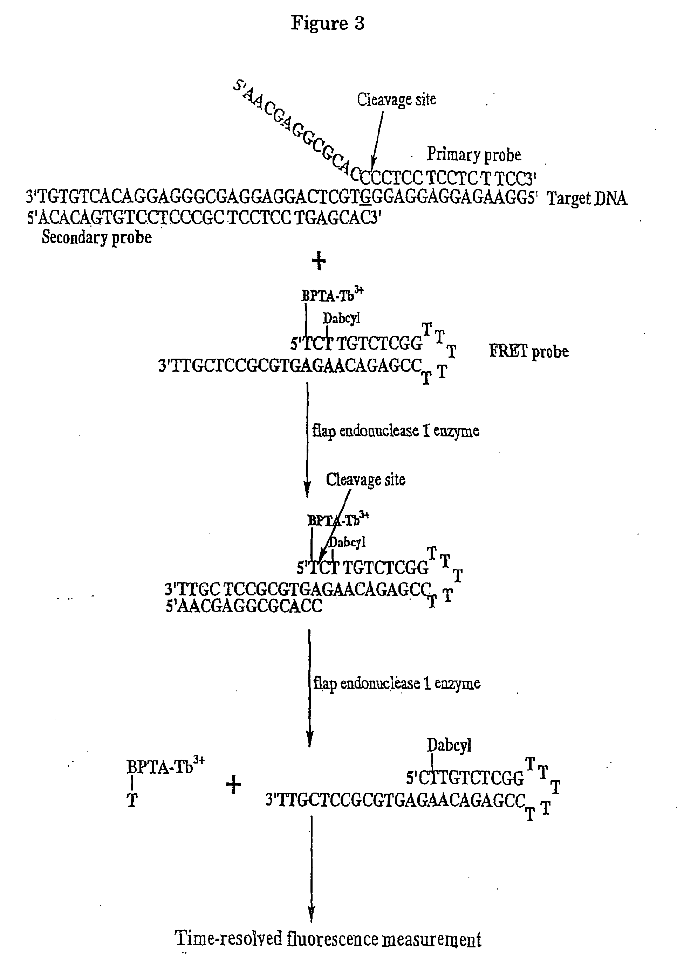

Using these reagents, an experiment was carried out according to the overview of the Invader method shown in Fig. 1. The overview of the concrete method is shown in Fig. 3. The underlined part of the target DNA in Fig. 3 indicates the location where SNPs are to be detected.

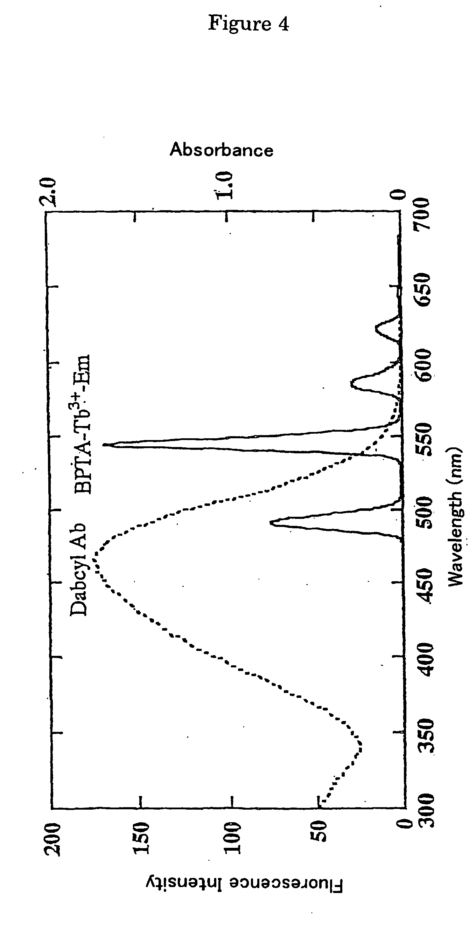

The absorbance spectrum of Dabcyl and the fluorescence luminescence spectrum of BPTA-Tb³⁺ were measured. The results are shown in Fig. 4. The horizontal axis in Fig. 4 is wavelength (nm) and the vertical axis on the left indicates fluorescence intensity and the vertical axis on the right indicates absorbance. The dotted line in Fig. 4 indicates the absorbance spectrum of Dabcyl and the solid line indicates the fluorescence luminescence spectrum of BPTA-Tb³⁺.

Overlapping the absorbance spectrum of Dabcyl and the fluorescence luminescence spectrum of BPTA-Tb³⁺, it is found that when the two come close to each other, the fluorescence luminescence of BPTA-Tb³⁺ is quenched by Dabcyl. On the contrary, when they are separated, BPTA-Tb³⁺ can emit strong fluorescence. In other words, in a FRET probe, because Dabcyl and BPTA-Tb³⁺ are within a close distance, the fluorescence of BPTA-Tb³⁺ is quenched. However, accompanying the reaction shown in Fig. 3, the BPTA-Tb³⁺ labeled nucleotide part is cleaved one after another and BPTA-Tb³⁺ comes to be separated from Dabcyl, and thereby the fluorescence of BPTA-Tb³⁺ gradually becomes stronger as the reaction progresses. By measuring this fluorescence by a time-resolved fluorescence assay method, an SNP in the target DNA can be analyzed.

On the contrary, in the case where a target DNA does not have an SNP, the cleavage reaction after the hybridization reaction of the first probe, the second probe and the target DNA does not occur, and because the hybridization reaction of a flap and a FRET probe and the cleavage reaction after the reaction do not occur, a large fluorescence increase of BPTA-Tb³⁺, which occurs when an SNP exists, is not measured, although a small fluorescence increase by non-specific reaction is measured.

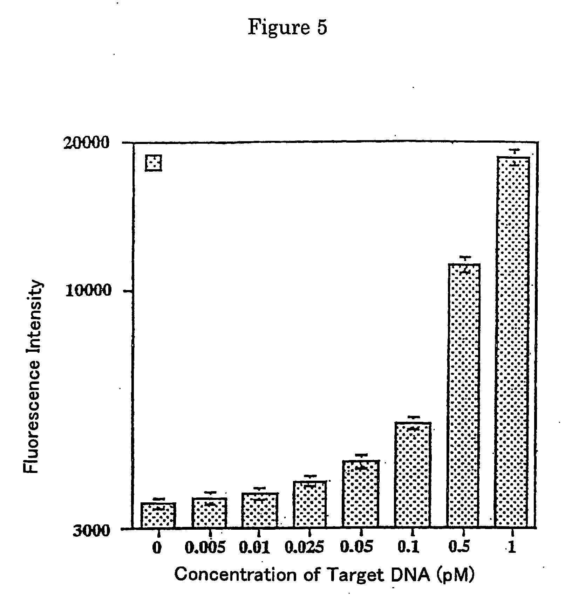

In Fig. 5, the change of fluorescence intensity accompanying the concentration changes of a target DNA is shown. The horizontal axis of Fig. 5 is the concentration (pM) of the target DNA and the vertical axis indicates fluorescence intensity. The point where the concentration of the target DNA is 0 indicates the background fluorescence intensity. According to this result, if the background + 2SD (standard deviation) is taken as a standard, the detection sensitivity is about 0.008 pM (8 x 10⁻¹⁵ M). This is more sensitive by about two orders of magnitude compared with a conventional method using an organic labeling agent.

As mentioned above, the method of the present invention, in which a rare earth fluorescent complex labeling agent is used as a luminescent dye and a fluorescent quencher labeling agent is used as a quenching substance, is extremely highly sensitive compared with a system using a conventional organic fluorescent labeling agent, and the ratio of fluorescence signal to noise after reaction is also large. This is because the fluorescence lifetime of a rare earth fluorescent complex labeling agent is very long and thus a time-resolved fluorescence assay method can be used for a long time with a fluorescent signal after reaction so that background noise, which has a short fluorescent lifetime, can be removed efficiently. In the case where a FRET probe composed of a conventional organic fluorescent dye is used, for example, in the case of a FRET probe composed of fluorescein and Cy3 (Proc. Natl. Acad. Sci. USA, 2000, 97, 8272), in the measurement of fluorescein fluorescence measurement after reaction, because Cy3 has a strong fluorescence at the maximum fluorescence wavelength of fluorescein, it creates large background noise. However, in the method of the present invention, because a time-resolved fluorescence assay method can be used, the fluorescence of Cy3, which has a short fluorescence lifetime, can be eliminated completely and the most sensitive measurement can be conducted. In addition, in the case where a europium fluorescent complex labeling agent as a rare earth fluorescent complex labeling agent or Cy5 as a fluorescent quencher labeling agent is used, a similar result is obtained.

A rare earth fluorescent complex labeling agent of the present invention is composed of a complex of rare earth elements, and various elements of lanthanoids may be included as the rare earth element, but samarium (Sm), europium (Eu), terbium (Tb) and dysprosium (Dy) are preferable. These rare earth elements can be in the zero valent form; however, a complex formed from elements in an ionic form, preferably in a trivalent cation form, is preferable.

Preferable ligands for the rare earth fluorescent complex labeling agent of the present invention include the ligands represented by the above-mentioned general formulae (1) to (6). The aryl group of the substituent group R in the ligands represented by the above-mentioned general formulae (1) to (6) may be a carbocyclic aromatic group such as a phenyl group and a naphthyl group, or a five to seven-membered heterocyclic aromatic group which contains one to three nitrogen atoms, oxygen atoms or sulfur atoms in the ring. These aromatic groups may be monocyclic, polycyclic or condensed cyclic systems. Preferable aryl groups include phenyl groups, thienyl groups, and pyridyl groups. The aryl group with the substituent group R has a substituent group and as the substituent group, a polar functional group such as an amino group, a hydroxyl group, a carboxyl group, a sulfonate group or an isothiocyanate group is preferable. Further, it may be an alkyl derivative or a halogenated derivative of these functional groups. Preferable substituent groups include amino groups, carboxyl groups, and isothiocyanate groups.

The substituent group R' in the ligands represented by the above-mentioned general formulae (1) to (6) can include an amino group, a hydroxyl group, a carboxyl group, a sulfonate group or an isothiocyanate group, but it is not limited to these, and a polar functional group is preferable. Further, it may be an alkyl derivative or a halogenated derivative of these functional groups. Preferable substituent groups include amino groups, carboxyl groups, and isothiocyanate groups.

Preferable examples of the fluorescent quencher labeling agent of the present invention include compounds represented by the above-mentioned general formulae (7) to (9).

The substituent groups R₁ and R₂ of the fluorescent quencher labeling agent of the present invention represented by the general formula (7) indicate that at least one of them is a linker group for immobilization on a carrier or a nucleic acid. When one of the substituent groups R₁ and R₂ is not a linker group, that one represents a hydrogen atom or an alkyl group, and preferably represents an alkyl group. The alkyl group in this invention is a linear or a branched alkyl group with a carbon number of 1 to 20, preferably a carbon number of 1 to 10, or more preferably a carbon number of 1 to 7. Preferable alkyl groups include methyl groups, ethyl groups, and isopropyl groups. In addition, the linker group for immobilization of the substituent group R₁, R₂ or R₃ in the general formulae (7) to (9) on a carrier or a nucleic acid may be any as long as it has an appropriate size for immobilization on a carrier or a nucleic acid and a reactive group for immobilization, and it can be arbitrarily selected depending on the kind of the carrier or the nucleic acid. For such a linker group, it is linked to a target carrier of nucleic acid by a linear alkylene group with a carbon number of 3 to 30, preferably o a carbon number of 5 to 20, and more preferably a carbon number of 5 to 15, in which one or more of carbon atoms of the alkylene group may be substituted by another atom such as an oxygen atom, a nitrogen atom or a sulfur atom, or one or more of carbon atoms, nitrogen atoms or sulfur atoms of the alkylene group maybe substituted by an oxo group (=O) , a halogen, a hydroxyl group, an amino group or an alkyl group.

A preparation method of a modified FRET nucleotide probe (a modified DNA oligonucleotide) used in the present invention can be carried out according to any of the nucleotide probe preparation methods which themselves are well known. For example, any oligonucleotide synthesis can be carried out by the phosphoramidite method using an automatic DNA synthesizer. The purification can be carried out by a reversed phase HPLC. In addition, the introduction of the fluorescent quencher labeling agent can be carried out according to a common method using a dye derivative such as Dabcyl-dT represented by the following chemical formula (10) 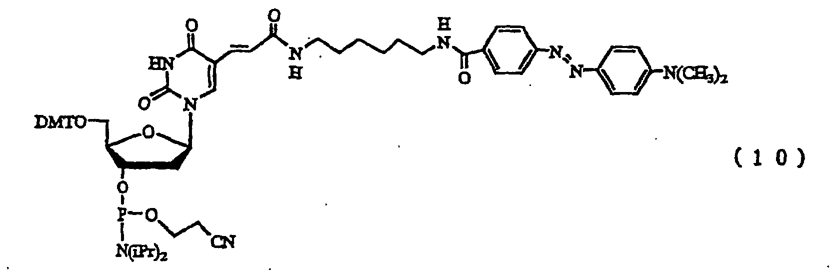 or TAMRA-dT represented by the following chemical formula (11) 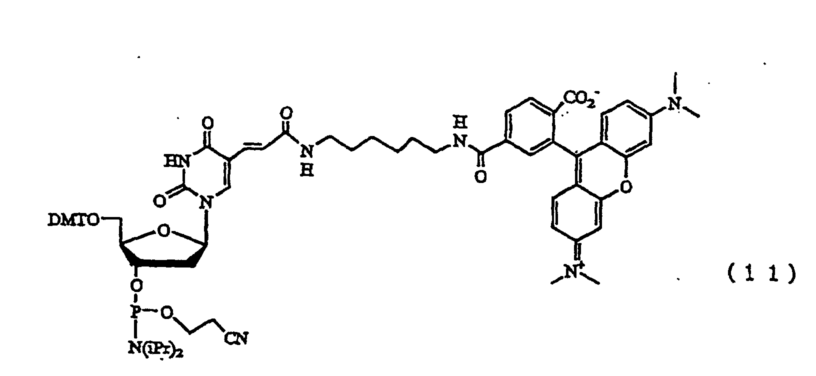 or Cy3-dC represented by the following chemical formula (12) 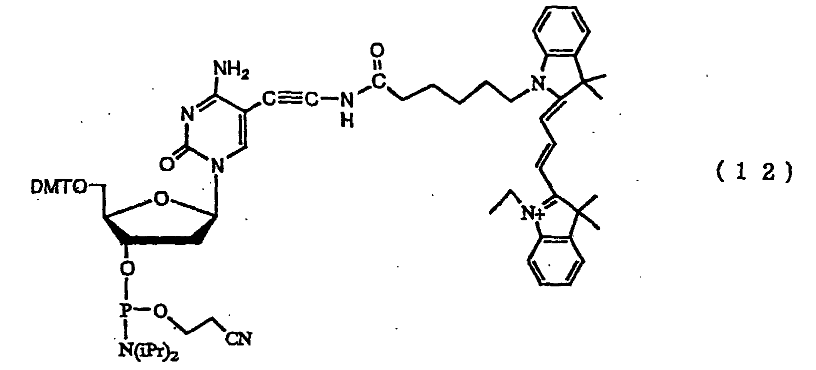 or Cy5-dC represented by the following chemical formula (13) 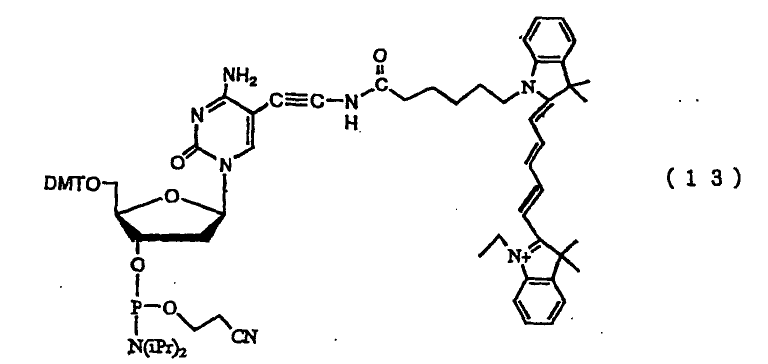 (in the chemical formula (10) to (13), DMTO represents a 4-monomethoxytrimethyl group and iPr represents a 2-propyl group).

Furthermore, the introduction of a rare earth fluorescent complex labeling agent can be carried out by the reactionwith a fluorescent complex labeling agent composed of any of the ligand represented by the chemical formulae (1) , (2), (3) , (4), (5) or (6), and a rare earth ion, using an oligonucleotide amino-modified at for example the 5' end, and the purification can be carried out by a reversed phase HPLC, an electrophoresis or a gel filtration chromatography.

The highly sensitive labeled probe of the present invention is a labeled probe which is used for an SNP analysis, in which the rare earth fluorescent complex labeling agent and the fluorescent quencher labeling agent of the present invention can be used in combination. Examples of the highly sensitive labeled probe of the present invention include a FRET probe in the Invader method, a probe with a hairpin structure in the molecular beacon method, or a TaqMan probe in the TaqMan PCR method, but it is not limited thereto.

Incidentally, all the contents described in the specifications, JP-A-2002-063960 and JP-A-2003-061958, are incorporated in this description.

### Examples

Hereunder, the present invention will be illustrated in more detail with reference to Examples, however, the present invention is not limited to these Examples.

### Example 1 (Production of terbium fluorescent labeling agent BPTA-Tb³⁺)

Terbium fluorescent labeling agent BPTA-Tb³⁺ is a complex composed of a terbium ion and an organic ligand BPTA, and BPTA and its N-sydroxysuccinateimide monoester (abbreviated as NHS-BPTA) was produced according to the method reported by the present inventors (J. Yuan, G. Wang, K. Majima, K. Matsumoto, Anal. Chem., 2001, 73, 1869).

### Example 2 (Preparation of DNA oligonucleotide)

All of the oligonucleotides shown in Fig. 3 were synthesized by the phosphoramidite method using an automatic DNA synthesizer. The purification was carried out by a reversed phase HPLC. The underlined nucleotide of the target DNA indicates an SNP site.

The preparation of FRET probe was carried out firstly by synthesizing a DNA oligonucleotide modified with a 5' amino group and Dabcyl by an automatic DNA synthesizer and further by labeling the 5' amino group using NHS-BPTA-Tb³⁺.

### Example 3 (SNP assay)

The SNP assay of a target DNA was carried out according to a conventional method (for example, the method described in the documents of Nature Biotechnology, 1999, 17, 292 and Proc. Natl. Acad. Sci. USA, 2000, 97, 8272). The principle is shown in Fig. 3. In other words, flap endonuclease 1 enzyme as a cleavage enzyme was added to a solution containing a reporter probe (Primary probe), an invader probe (Secondary probe), a target DNA and a FRET probe, and after incubation at 63°C for 4 hours, a time-resolved fluorescence assay was carried out.

The measurement device was ARVO 1420 (Wallac). The measurement condition was as follows: the delay time was 0.4 millisecond, the window time was 0.4 millisecond, the cycle time was 1.0 millisecond, the excitation wavelength was 320 nm and the measurement wavelength was 545 nm.

### Example 4 Application of BPTA for Molecular beacon method (1)

To apply BPTA for the detection of a hybridization signal using the molecular beacon method, the following experiment was conducted.

As a target sequence desired to be detected, an oligonucleotide having the sequence of 5' - GCTGGAGGGATGATACTTTGCA- 3' was synthesized.

For a probe used as a molecular beacon, an oligonucleotide having the sequence of 5' -CCAAGCGCAAAGTATCATCCCTCCAGGCTTGG-3', to which BPTA was bound at the 5' end via a spacer of (CH₂)₆ and DABCYL (4-(dimethylamino)azobenzene-4'-sulfonyl) group was bound at the 3' end as a quencher, was prepared. The 6 nucleotide residues of the 5' end side and the 3' end side in this sequence are complementary, and the probe is designed so that in the case where the sequence between the above sequences does not hybridize to a complementary nucleotide sequence, a stem structure is formed, BPTA and DABCYL are close together in the molecule, and the light in the wavelength range by which BPTA is excited is absorbed by the DABCYL group, and thus the net result is that BPTA derived fluorescence is reduced.

In addition, as a non-target oligonucleotide, the sequence 5' -CCATGGTGTCTGTTTAAGGTTGCT- 3' was used.

The reaction was carried out using oligonucleotides comprising 0.2 µM of the above-mentioned molecular beacon and 0.6 µM of the target sequence (or non-target) in 50 µL of 15 mM Tris-HCl (pH 7.0) buffer solution containing 2 mM MgCl₂ and 0.6 µM TbCl₃ at room temperature for 5 minutes. After the reaction, BPTA derived fluorescence as a hybridization signal was measured using an ARVO™ SX 1420 Multilabel Counter manufactured by WALLAC. In addition, a reaction solution with the oligonucleotide of the target sequence (or non-target) not added was used as the blank of reaction.

The results are shown in Table I. A significantly higher signal was obtained with the target sequence than with the non-target sequence.

**Table I**

| Sequence | Fluorescence intensity |
|---|---|
| Target sequence | 803,760 |
| Non-target sequence | 95,728 |

### Example 5 Application of BPTA for Molecular beacon method (2)

By applying the quenching capability of DABCYL used in Example 4, single nucleotide difference of DNA was detected using oligo DNA labeled with BPTA.

In this example, 4 kinds of oligonucleotides having the nucleotide sequences shown in Table II were synthesized as the target DNAs.

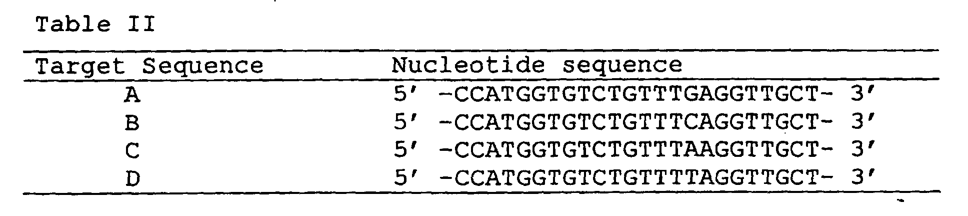

These oligonucleotides were designed to be different in sequence only for the 16th nucleotide from the 5' end and to be the identical for rest of the sequence.
As a BPTA labeled probe, an oligonucleotide having the sequence of 5' -AGCAACCTCAAACAGACACCATGG- 3', to which BPTA was bound at the 5' end via a spacer of (CH₂)₆, was prepared. This sequence is completely complementary to the target sequence A in Table II. Thus, under an appropriate condition, hybridization specifically to the target sequence A is possible, as opposed to the target sequences B to D in which one nucleotide mismatch exists.

Meanwhile, an oligonucleotide having the sequence of 5' - GGTGTCTGTTTGAGGTTGCT- 3', to which DABCYL was bound at the 3' end was prepared (oligonucleotide for quenching). Because this sequence is complementary to the sequence of the 1st to 20th nucleotides from the 5' end in the above-mentioned BPTA labeled probe and hybridizable, it is expected to quench fluorescence derived from free BPTA labeled probe. For this reason, single nucleotide difference can be distinguished by the difference in signal fluorescence intensity between the target sequence A (completely complementary sequence) and B to D (sequences with single nucleotide difference).

The reaction was carried out using 1 µM of the above-mentioned BPTA labeled probe, 2 µM of oligonucleotide having the target sequence and 2 µM of oligonucleotide for quenching in 50 µL of 15 mM Tris-HCl (pH 7.0) buffer solution containing 2 mM MgCl₂ and 3 µM TbCl₃ at room temperature for 30 minutes. After the reaction, BPTA derived fluorescence as a hybridization signal was measured using an ARVO™ SX 1420 Multilabel Counter manufactured by WALLAC. In addition, a reaction solution with the Oligonucleotide of the target sequence (or non-target) not added was used as the blank of reaction.

The results are shown in Table III.

**Table III**

| Target Sequence | Fluorescence intensity |
|---|---|
| A | 5,526,292 |
| B | 344,573 |
| C | 432,303 |
| D | 496,235 |

As a result, the signal for the completely complementary sequence was significantly higher than the signals for the sequences with a single nucleotide difference. In other words, a single nucleotide difference in nucleotide sequence could be distinguished.

### Industrial Applicability

The present invention provides a highly sensitive nucleotide SNP analysis method based on a time-resolved fluorescence assay with the use of a FRET probe composed of a rare earth fluorescent complex labeling agent and a fluorescent quencher labeling agent. According to the method of the present invention, an analysis becomes possible at a target DNA concentration of 0.008 pM (8 x 10⁻¹⁵ M), and analysis of a large amount of SNPs becomes possible with an extremely small amount of a target DNA.

In addition, according to the method of the present invention, because a background noise having a short fluorescent lifetime can be eliminated effectively, the fluorescent signal/noise ratio after reaction is large and analysis accuracy can be improved significantly.

## Claims

1. An SNP analysis method by the Invader method, in which a FRET probe containing a fluorescent dye and a quenching substance is used, **characterized by** using a rare earth fluorescent complex labeling agent as a luminescent dye in the FRET probe.

2. The method according to Claim 1, wherein the rare earth fluorescent complex labeling agent comprises any one of the ligands represented by the following general formulae (1) to (6) as a ligand, 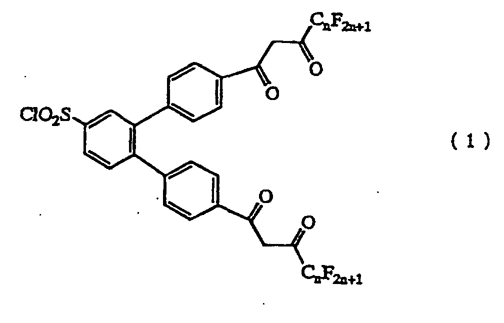 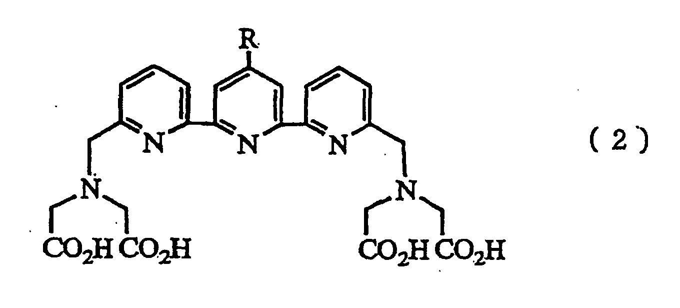 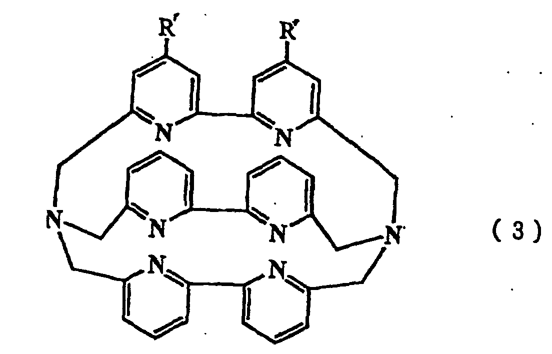 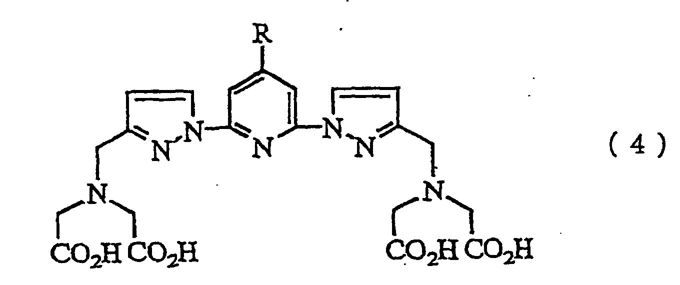 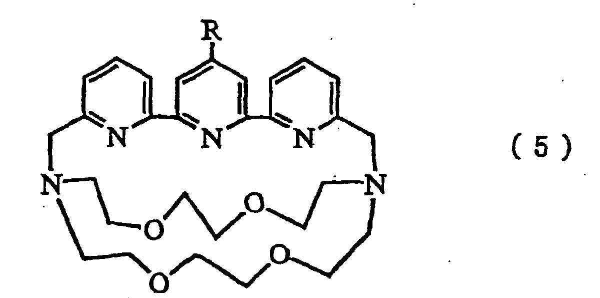 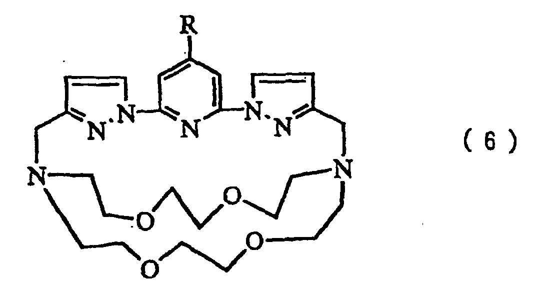 (in the formulae (1) to (6), n represents an integer of 1 to 4, R represents an allyl group having a substituent group and R' represents an amino group, a hydroxyl group, a carboxyl group, a sulfonate group or an isothiocyanate group).

3. The method according to Claim 1 or 2, wherein a rare earth element in the rare earth fluorescent complex labeling agent is samarium (Sm) , europium (Eu) , terbium (Tb) or dysprosium (Dy).

4. The method according to any one of Claims 1 to 3, wherein the quenching substance in the FRET probe is a fluorescent quencher labeling agent.

5. The method according to Claim 4, wherein the fluorescent quencher labeling agent is a substance represented by the following general formulae (7) to (9), 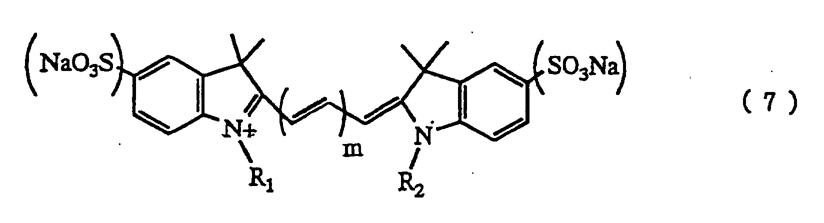 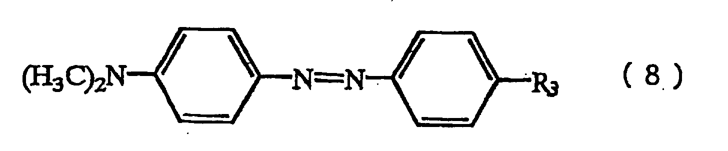 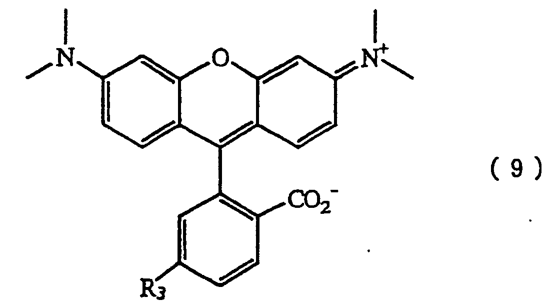 (In the formulae (7) to (9), m represents an integer of 1 to 4, either R₁ or R₂ represents a linker group for immobilization on a carrier or a nucleic acid, the other represents a hydrogen atom or an alkyl group, and R₃ represents a linker group for immobilization on a carrier or a nucleic acid).

6. The method according to any one of Claims 1 to 5, wherein the SNP analysis method is **characterized by** measuring fluorescence luminescence by a time-resolved fluorescence assay method.

7. A composition for controlling fluorescence luminescence comprising a combination of a rare earth fluorescent complex labeling agent composed of one or more of rare earth fluorescent complex labeling agents containing at least one ligand represented by the following general formulae (1) to (6) as a ligand, 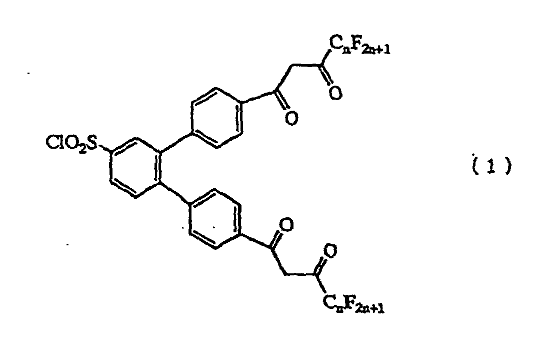 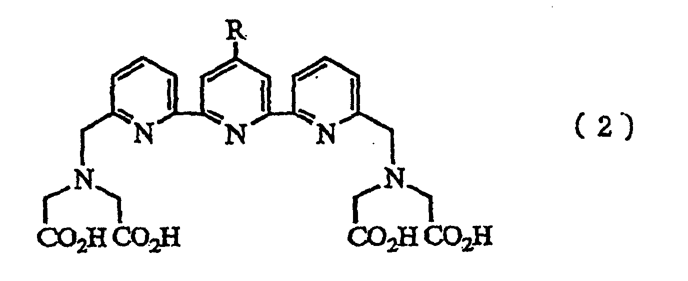 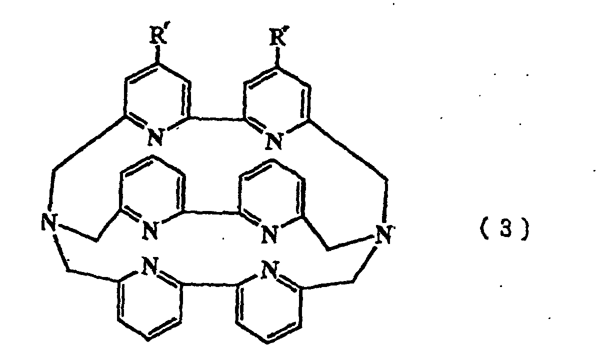 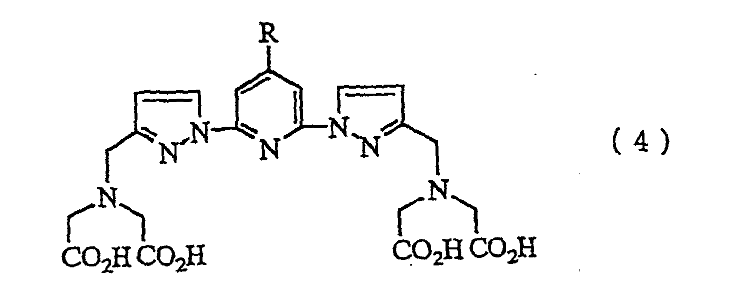 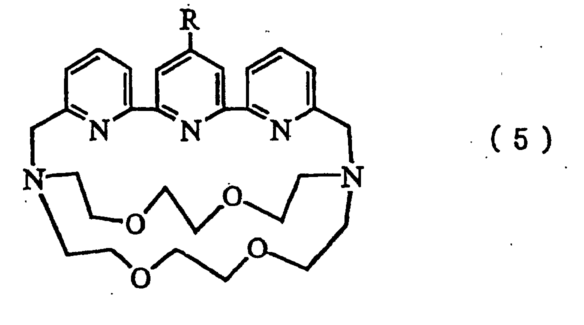 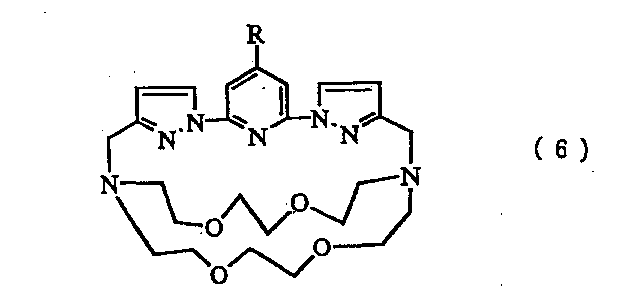 (in the formulae (1) to (6), n represents an integer of 1 to 4, R represents an allyl group having a substituent group and R' represents an amino group, a hydroxyl group, a carboxyl group, a sulfonate group or an isothiocyanate group) , and a fluorescent quencher labeling agent composed of one or more of substances represented by the following general formulae (7) to (9). 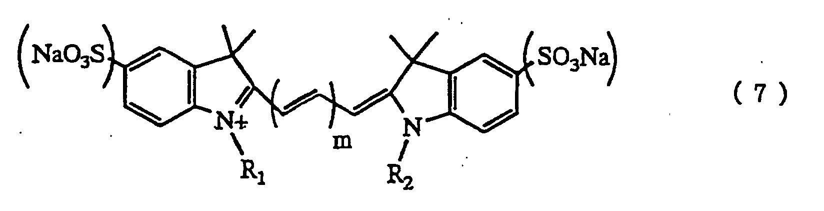 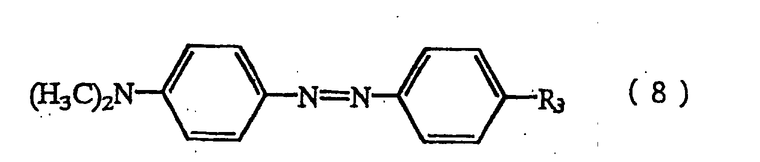 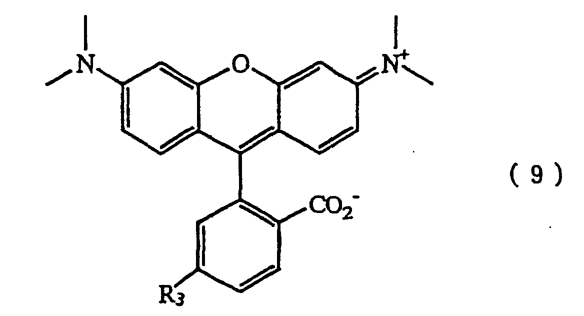 (in the formulae (7) to (9), m represents an integer of 1 to 4, either R₁ or R₂ represents a linker group for immobilization on a carrier or a nucleic acid, the other represents a hydrogen atom or an alkyl group, and R₃ represents a linker group for immobilization on a carrier or a nucleic acid).

8. The composition for controlling fluorescence luminescence according to Claim 7, wherein a rare earth element in the rare earth fluorescent complex labeling agent is samarium (Sm), europium (Eu), terbium (Tb) or dysprosium (Dy).

9. The composition for controlling fluorescence luminescence according to Claim 7 or 8, wherein the composition for controlling fluorescence luminescence in an SNP analysis method by the Invader method, in which a FRET probe containing a fluorescent dye and a quenching substance is used, is for an SNP analysis **characterized by** using a rare earth fluorescent complex labeling agent as the luminescent dye in the FRET probe.

10. Ahighly sensitive labeledprobe for an SNP analysis containing a luminescent dye and a quenching substance **characterized by** using a rare earth fluorescent complex labeling agent as the luminescent dye and using a fluorescent quencher labeling agent as the quenching substance.

11. The highly sensitive labeled probe according to Claim 10, comprising a combination of a rare earth fluorescent complex labeling agent and a fluorescent quencher labeling agent, wherein the rare earth fluorescent complex labeling agent and the fluorescent quencher labeling agent are according to any one of Claims 7 to 9.

12. The highly sensitive labeled probe according to Claim 10 or 11, wherein the highly sensitive labeled probe is a FRET probe.

13. The highly sensitive labeled probe according to Claim 10 or 11, wherein the highly sensitive labeled probe is a molecular beacon probe.

14. A kit for an SNP analysis, comprising the highly sensitive labeled probe according to any one of Claims 10 to 13.
